# FASCICULE DE BREVET EUROPEEN

(11) **EP 1 144 436 B1**
(45) Date de publication et mention de la délivrance du brevet: **07.04.2004**
(21) Numéro de dépôt: 00900552.1
(22) Date de dépôt: 11.01.2000
(51) Int. Cl.: C07K 5/078, C07D 237/04

(54) **PROCEDE DE PREPARATION DE COMPOSES BICYCLIQUES ET L'APPLICATION DE CE PROCEDE POUR LA PREPARATION D'UN COMPOSE INHIBITEUR DE ICE**
VERFAHREN ZUR HERSTELLUNG VON BIOZYKLISCHEN VERBINDUNGEN UND DEREN VERWENDUNG ZUR HERSTELLUNG VON ICE INHIBITOREN
METHOD FOR PREPARING BICYCLIC COMPOUNDS AND USE OF SAID METHOD FOR PREPARING AN INTERLEUKIN-1(Beta) CONVERSION ENZYME (ICE) INHIBITOR

(30) Priorité: 12.01.1999 FR 9900227
(43) Date de publication de la demande: 17.10.2001
(73) Titulaire: Aventis Pharma S.A., 92160 Antony (FR)
(72) Inventeur: CROCQ, Véronique, F-21000 Dijon (FR); ROUSSEL, Patrick, F-94320 Thiais (FR)
(74) Mandataire: Rousseau, Pierrick Edouard
(86) Numéro de dépôt international: PCT/FR2000/000041
(87) Numéro de publication internationale: WO 2000/042061

(56) Documents cités:
- EP-A- 0 094 095
- ATTWOOD M R ET AL: "THE DESIGN AND SYNTHESIS OF THE ANGIOTENSIN CONVERTING ENZYME INHIBITOR CILAZAPRIL AND RELATED BICYCLIC COMPOUNDS" JOURNAL OF THE CHEMICAL SOCIETY, PERKIN TRANSACTIONS 1, 1 janvier 1986 (1986-01-01), pages 1011-1019, XP000196065 ISSN: 0300-922X
- ATTWOOD M R ET AL: "NEW POTENT INHIBITORS OF ANGIOTENSIN CONVERTING ENZYME" FEBS LETTERS, vol. 165, no. 2, 1 janvier 1984 (1984-01-01), pages 201-206, XP002047481 ISSN: 0014-5793

## Description

La présente invention a pour objet un nouveau procédé de préparation de composés bicycliques et l'application de ce procédé comme étape intermédiaire pour la préparation d'un composé inhibiteur de l'enzyme de conversion de l'interleukine 1-bêta (ICE).

Le composé de formule (I) telle que définie plus bas, dans laquelle R représente un radical terbutyle et l'amine est protégée sous la forme de phtalimido, est décrit dans le brevet EP 94095. Ce composé de formule (I) est également utilisé pour la préparation du composé de formule (V) ayant une activité inhibitrice de l'enzyme de conversion de l'interleukine 1-bêta décrit dans la demande internationale WO 97/22619.

Un des objectifs de l'invention est de trouver un nouveau procédé d'obtention des composés de formule (I).

L'invention a donc pour objet un procédé de préparation des composés de formule (I) 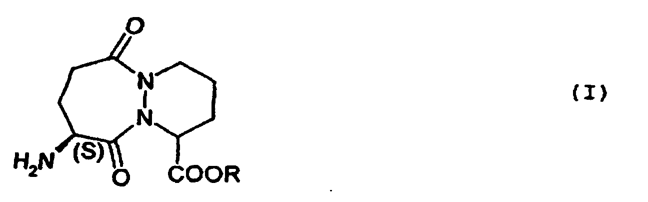 dans laquelle R représente un atome d'hydrogène, un radical alkyle, aryle ou aralkyle renfermant jusqu'à 18 atomes de carbone et la fonction amine peut être libre ou protégée, à partir d'un composé de formule (IA) 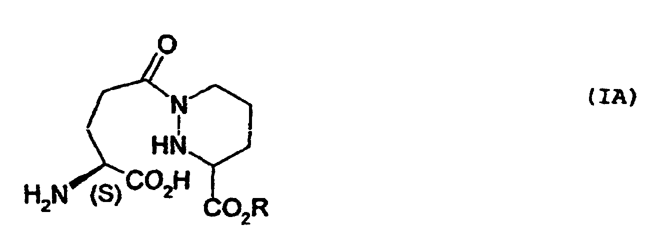 dans laquelle R a la même signification que précédemment et la fonction amine peut être libre ou protégée,
caractérisé en ce que la cyclisation s'effectue en milieu basique et en présence :
- d'un dérivé d'acide phosphonique de formule (P1) :

   P(=Z) (X¹) (X²) (X³)

   dans lequel Z est un atome de soufre ou d'oxygène, X¹ représente un atome d'halogène, X² et X³ identiques ou différents représentent un atome d'halogène, un radical alkyloxy renfermant de 1 à 6 atomes de carbone, un radical aryloxy renfermant de 6 à 12 atomes de carbones ou un radical arylalkyloxy renfermant de 7 à 15 atomes de carbone,
- ou d'un trimère de formule (P2) : 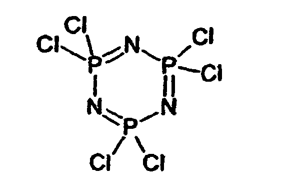 R représente de préférence un atome d'hydrogène, un radical méthyle, éthyle, propyle, isopropyle, n-butyle, isobutyle, tertbutyle, benzyle, phényle ou naphtyle et tout particulièrement mèchyle, éthyle et tertbutyle.

Lorsque la fonction amine est protégée, la protection peut se faire selon les méthodes classiques connues de l'homme du métier.

La fonction amine peut être protégée sous la forme d'un radical -NR¹R² dans lequel
- soit R¹ représente un radical 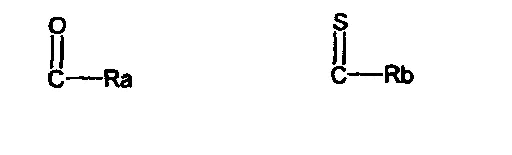 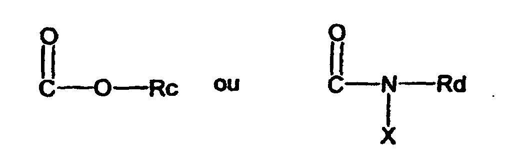 Ra, Rb, Rc et Rd représentant un radical alkyle ou aryle renfermant jusqu'à 18 atomes de carbone ou un radical mono ou polycyclique renfermant un ou plusieurs hétéroatomes, X représentant un atome d'hydrogène, un radical alkyle renfermant jusqu'à 8 atomes de carbone ou un radical aryle renfermant jusqu'à 14 atomes de carbone, et R² représente un atome d'hydrogène,
- soit R¹ et R² forment ensemble un radical mono ou polycyclique renfermant 1 ou plusieurs hétéroatomes. L'amine peut ainsi être protégée sous la forme d'un phtalimido 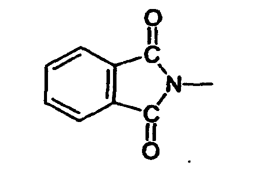 ou encore sous la forme du radical

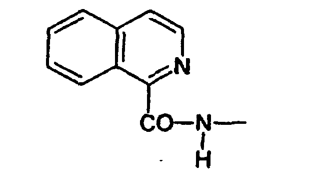

De préférence, l'amine est protégée sous la forme d'un phtalimido.

Parmi les dérivés d'acide phosphonique de formule P(=Z) (X¹) (X²) (X³), il s'agit en particulier des dérivés suivants :
(Cl)P(O)(Ph)₂, (Cl)₂P(O)(OPh)₂, (Cl)P(O)(OEt)₂, (Cl)₂P(O)(OEt)₂, POCl₃, POBr₃ et P(S)Cl₃.

La réaction de cyclisation s'effectue de préférence en présence de POCl₃ ou de POBr₃ et la base est notamment une base organique, par exemple la triéthylamine, la pyridine ou la 2,6-lutidine.

L'invention a plus particulièrement pour objet le procédé tel que décrit précédemment dans lequel l'acide trihalogénophosphonique est le POCl₃.

L'invention a plus particulièrement pour objet le procédé tel que décrit précédemment dans lequel l'acide trihalogénophosphonique est le POBr₃.

L'invention a plus particulièrement pour objet le procédé tel que décrit précédemment dans lequel la base est choisie parmi la pyridine ou la 2,6-lutidine.

L'invention a plus particulièrement pour objet le procédé tel que défini précédemment caractérisé en ce que la température de cyclisation est comprise entre 70 et 80 C°.

L'invention a plus particulièrement pour objet le procédé tel que défini précédemment caractérisé en ce que le solvant est le dichloroéthane.

Le composé de formule (IA) se trouve sous la forme d'un mélange de diastéréoisomères SS et SR ou sous la forme du diastéréoisomère SR.

Le composé de formule (I) se trouve sous la forme d'un mélange de diastéréoisomères SS et SR ou sous la forme du diastéréoisomère SR.

L'invention a également pour objet un procédé de préparation du composé de formule (I) sous forme racémique ou optiquement actif (Iopt), comprenant le procédé de cyclisation tel que décrit plus haut, et caractérisé en ce qu'il comprend les étapes successives suivantes :
a) On soumet un composé de formule (II) 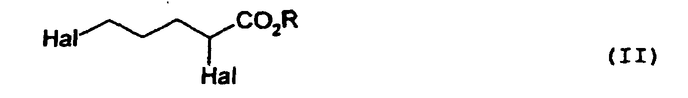 dans laquelle R est tel que défini précédemment et Hal représente un atome d'halogène, à l'action d'un composé de formule (III) 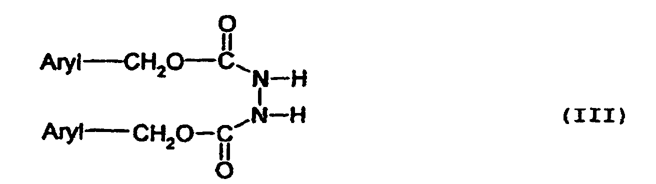 dans laquelle Aryl représente un radical aryle renfermant jusqu'à 14 atomes de carbone, pour obtenir le composé de formule (IV) 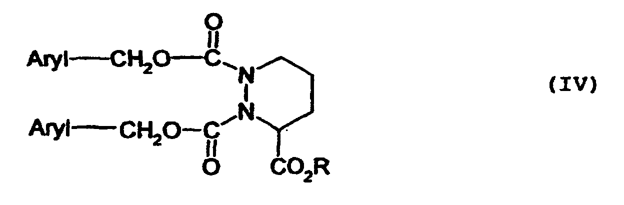 sous la forme d'un mélange de stéréoisomères S et R,
b) on soumet le composé de formule (IV) à l'action d'un anhydride de formule (F) 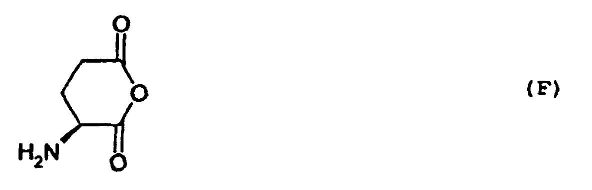 la fonction amine étant sous forme protégée ou non protégée, tout en effectuant une déprotection par hydrogènolyse, afin d'obtenir le composé de formule (IA) 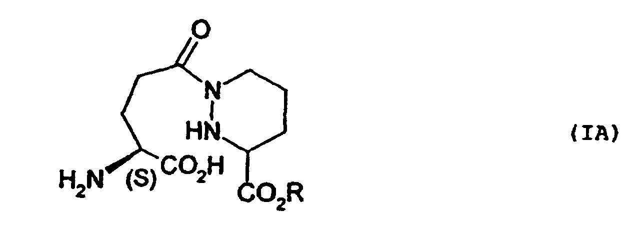 tel que défini plus haut,
c) on soumet le composé de formule (IA) à l'action d'un dérivé d'acide phosphonique (P1) ou (P2) tel que défini plus haut, en présence d'une base, afin d'obtenir le composé de formule (I) tel que défini plus haut 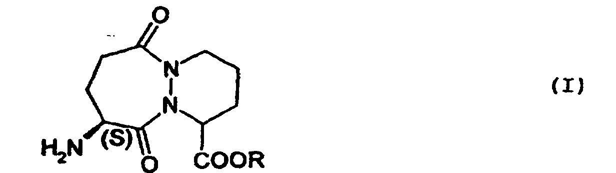
d) le cas échéant, on déprotège la fonction amine pour obtenir le composé de formule (I) dans lequel la fonction amine est non protégée,
e) on soumet, le cas échéant, le composé de formule (I) sous la forme SS + SR ou SR à l'action d'un agent de déracémisation et/ou épimérisation afin d'obtenir le composé de formule (Iopt) correspondant au diastéréoisomère SS, 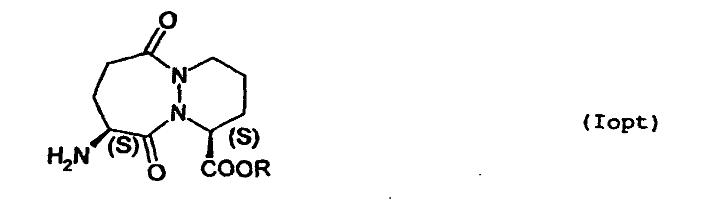
f) le cas échéant, on déprotège la fonction amine pour obtenir le composé de formule (Iopt)dans lequel la fonction amine est non protégée.

Dans un mode de réalisation préférée :
- Hal représente un atome de chlore ;
- R représente un radical alkyle renfermant de 1 à 4 atomes de carbone ;
- Aryl représente un radical phényle ou naphtyle,
- Le composé (F) est l'anhydride N-phtaloyl-L- glutamique 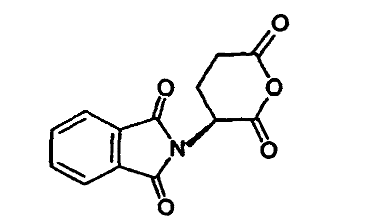
- La fonction amine des composés de formules (IA), (I) ou (Iopt) est sous forme protégée et notamment sous forme phtalimido ;
- La réaction entre les composés de formule (II) et les composés de formule (III) a lieu en présence d'une base par exemple en présence d'un carbonate alcalin comme le carbonate de potassium ;
- La déprotection par hydrogénolyse s'effectue selon les conditions classiques connues de l'homme du métier, par exemple l'agent d'hydrogénolyse est l'hydrogène en présence de palladium sur charbon ;
- L'agent de déracémisation et/ou d'épimérisation est une base, plus spécialement une base forte, par exemple un alcoolate alcalin ou alcalinoterreux comme le méthylate de sodium ou de potassium, le terbutylate de sodium ou de potassium ou une amine lithiée comme la LDA ;
- L'action d'un agent de déprotection de l'amine peut s'effectuer notamment par action d'une hydrazine.

L'invention a tout particulièrement pour objet le procédé tel que défini précédemment dans lequel (F) est l'anhydride de l'acide phtaloyl glutamique

L'invention a tout particulièrement pour objet le procédé tel que défini précédemment dans lequel la fonction amine des composés de formules (IA), (I) ou (Iopt) est protégée sous forme d'un groupement phtalimido.

L'invention a tout particulièrement pour objet le procédé tel que défini précédemment dans lequel, au sein des composés de formule (II), (IV), (IA), (I) et (Iopt), R est un radical méthyle, éthyle ou tertbutyle.

L'invention a tout particulièrement pour objet le procédé tel que défini précédemment dans lequel le composé de formule (I) est le 9-(1,3-dihydro-1,3-dioxo-2H-isoindol-2-yl)-3,4,7,8,9,10-hexahydro-6,10-dioxo-6H-pyridazino-[1,2-a][1,2]diazepine-1-carboxylate d'éthyle :

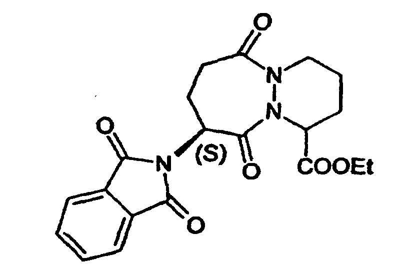

L'invention a tout particulièrement pour objet le procédé tel que défini précédemment dans lequel le composé de formule (Iopt) est le
- (1S-cis)-9-(1,3-dihydro-1,3-dioxo-2H-isoindol-2-yl)-3,4,7,8,9,10-hexahydro-6,10-dioxo-6H-pyridazino-[1,2-a][1,2]diazepine-1-carboxylate d'éthyle :

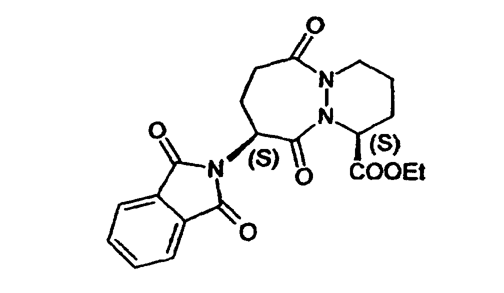

Les composés de formule (I) peuvent être de façon générale utilisés pour la synthèse de médicaments comme il est indiqué dans le brevet EP 94095. Les composés de formules (II) et (III) et (F) sont connus et peuvent être préparés selon la méthode expérimentale décrite plus bas.

L'invention a également pour objet l'application du procédé tel que défini ci-dessus comme étape intermédiaire pour la préparation d'un composé de formule (V) 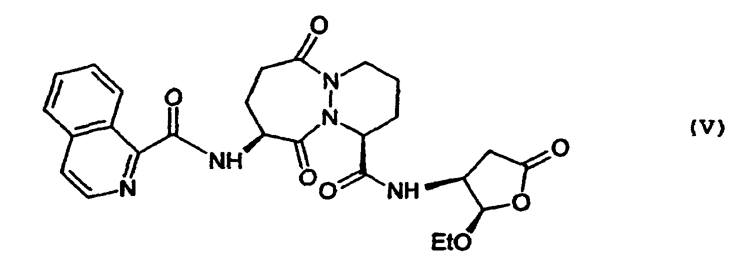 via le composé de formule (Iopt) tel que défini précédemment, caractérisée en ce que ce procédé comporte les étapes du procédé de préparation des composés de formule (Iopt) à partir des composés de formule (II) telles que définies précédemment.

L'invention a également pour objet l'application telle que définie ci-dessus, caractérisé en ce que le composé de formule (Iopt) est le (1S-cis)-9-(1,3-dihydro-1,3-dioxo-2H-isoindol-2-yl)-3,4,7,8,9,10-hexahydro-6,10-dioxo-6H-pyridazino-[1,2-a][1,2]diazepine-1-carboxylate d'éthyle

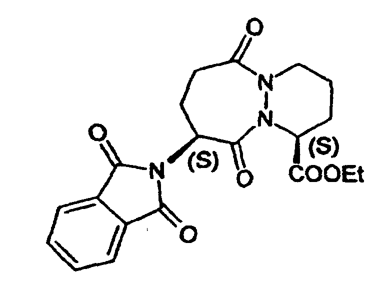

L'invention a aussi pour objet l'application du procédé tel que défini précédemment comme étape intermédiaire du procédé global de préparation des composés de formule (I) et (Iopt) tels que définis précédemment.

L'invention a enfin pour objet, à titre de composé intermédiaire, le composé de formule (IA) tel que défini précédemment.

Les exemples suivants illustrent l'invention sans toutefois la limiter.

### Préparation 1

### Préparation du 1,2-hydrazinecarboxylate de bis(phénylméthyle)

On place sous azote 1,5 litre de méthanol et 25 g d'hydrazine monohydrate à 80%. On refroidit à 0°C et introduit ensuite 75 g de chloroformiate de benzyle et une solution de 93 g de carbonate de sodium dans 1100 ml d'eau déminéralisée. On maintient le mélange réactionnel pendant 1 heure à 0°C, essore et lave par déplacement avec un mélange de 100 ml de méthanol et 100 ml d'eau, puis on lave par déplacement avec 500 ml d'eau à 0 C°. On sèche et obtient 107,6 g de produit recherché.

### Préparation 2

### Préparation de l'anhydride N-phtaloyl-L-glutamique D (+)

### 2-tétrahydro-2,6-dioxo-2H-pyran-3-yl-1H-isoindole-1,3(2H)-dione (R)

### Stade a : Acide N-phtaloyl-L-glutamique

### Acide 2-(1,3-dihydro-1,3-dioxo-2H-isoindole-2-yl)-pentanedioique (2S)

A une solution de 14,4 g de carbonate de sodium dans 180 ml d'eau on ajoute 10 g d'acide L-glutamique puis 16 g de N-carbethoxyphtalimide (réactif de nefkens, commercial). On agite à température ambiante pendant 2 heures puis extrait avec de l'acétate d'éthyle. On évapore la phase organique sous pression réduite jusqu'à obtention d'un extrait sec et obtient 2,74 g de produit brut. On effectue un lavage avec du bicarbonate de sodium, puis après retour à l'acide et extraction à l'acétate d'éthyle, on isole 370 mg de produit attendu et de H₂N-CO₂Et. Par ailleurs, la phase aqueuse est amenée au pH=2 avec de l'acide chlorhydrique à 36% à une température inférieure à 5°C puis on extrait à l'acétate d'éthyle, lave avec une solution saturée en chlorure de sodium, sèche, filtre et concentre sous pression réduite jusqu'à obtention de 22,7 g de produit attendu sous la forme d'une huile.
Spectre de masse (M-H)⁻ = 276⁻
Infrarouge (Nujol) :
1775 cm⁻¹(m), 1720 cm⁻¹ (F, complexe) : CO
1611 cm⁻¹ : Aromatique

### Stade b :

Au produit obtenu au stade a), on ajoute 160 ml de tétrahydrofurane et ajoute goutte à goutte en 30 minutes 18,6 g dé DCC (1,3-Dicyclohexyl-carbodiimide) en solution dans 55 ml de tétrahydrofurane. On agite 1 heure à 15-17°C, puis on filtre, rince avec du tétrahydrofurane, évapore sous pression réduite jusqu'à obtention d'un extrait sec qui est repris dans de l'oxyde d'isopropyle. Après 30 minutes d'agitation, on filtre lave et sèche. On obtient 14,98 g de produit attendu.
α_{D} = -52,63
¹H RMN (DMSO) 2,12 (m, 1H) ; 2,61 (m, 1H) ; 2,98 (dm, 1H) ; 3,16 (ddd, 1H) ; 5,48 (dd, 1H) ; 7,82 (m, >4H)

### Exemple 1 : (1S-cis)-9-(1,3-dihydro-1,3-dioxo-2H-isoindol-2-yl)-3,4,7,8,9,10-hexahydro-6,10-dioxo-6H-pyridazino-[1,2-a][1,2]diazepine-1-carboxylate d'éthyle.

### Stade a : préparation de l'acide 2,5-dibromopentanoique

On ajoute 39 ml de brome dans un mélange de 106 g d'acide 5-bromopentanoïque et de 1 ml de tribromure de phosphore. On porte le mélange réactionnel à 70-80°C pendant 16 h 30. On amène le milieu réactionnel à 100°C pendant 15 minutes et laisse revenir à la température ambiante. On obtient 147 g de produit recherché.

### Stade b : Préparation du 2,5-dibromopentanoate d'éthyle

On ajoute 24,37 g de chlorure d'oxalyle dans un mélange renfermant 50 g de l'acide préparé au stade précédent, 15 gouttes de diméthylformamide et 300 ml de dichlorométhane. On maintient le mélange réactionnel sous agitation, à la température ambiante, jusqu'à ce que la réaction soit complète. On refroidit le mélange réactionnel à 10°C et ajoute 50 ml d'alcool éthylique. On agite pendant 30 minutes à 10°C, laisse revenir à la température ambiante et agite 3 heures à température ambiante. On amène à sec et obtient le produit recherché.

### Stade c : Cyclisation

### Préparation de (S)-tétrahydro-1,2,3-pyridazinetricarboxylate de 3-éthyle-1,2-bis(phénylméthyle) et (R)-tétrahydro-1,2,3-pyridazinetricarboxylate de 3-éthyle-1,2-bis(phénylméthyle) .

On introduit à 20-25°C, une suspension de 12,1 g de 2,5-dibromopentanoate d'éthyle (stade b) dans 50 ml de diglyme dans une suspension renfermant 10,42 g de 1,2-hydrazinecarboxylate de bis(phénylméthyle) (préparation 1), 65 ml de diglyme et 8,26 g de carbonate de potassium. On chauffe à 90°C la suspension obtenue et maintient l'agitation pendant 48 heures. On refroidit à 20°C, verse dans une solution renfermant 50 ml d'acide chlorhydrique 2N et 150 ml d'un mélange d'eau et de glace. On extrait à l'acétate d'éthyle, lave à l'eau et sèche. On filtre, rince à l'acétate d'éthyle et sèche. Enfin, on purifie le produit brut par chromatographie sur silice en éluant avec un mélange heptane/acétate d'éthyle 40/20 et obtient 10,71 g de produit recherché.

### Stade d : Acylation et hydrogénolyse

### Préparation de l'acide α, (1S)-[3-oxo-3-(tétrahydro-3-éthoxycarbonyl-1(2H)-pyridazinyl)propyl]-1,3-dihydro-1,3-dioxo-2H-isoindole-2-acétique

On place sous pression d'hydrogène (1,3 bar) pendant 24 heures le mélange constitué de 15g de tétrahydro-1,2,3-pyridazinetricarboxylate de 3-éthyle-1,2-bis(phénylméthyle) sous la forme d'un mélange R + S tel que préparé au stade c 150 ml de tétrahydrofurane, 2,5 g de palladium sur carbone (10%) et 9,08 g d'anhydride de l'acide phtaloyl glutamique tel que préparé selon la préparation 2. Après filtration, on évapore sous pression réduite jusqu'à obtention d'un extrait sec que l'on reprend avec 100 ml d'acétate d'éthyle et 150 ml d'une solution saturée de bicarbonate de sodium. On extrait à 3 reprises et acidifie la solution de bicarbonate à pH=3 avec de l'acide chlorhydrique à 36%. On extrait à 3 reprises avec du dichlorométhane et lave avec de l'eau. On obtient 13,16 g de produit brut que l'on purifie par chromatographie sur silice en éluant avec le mélange toluène/acétate d'éthyle/acide acétique 20/80/1,5 pour obtenir 12,7 g de produit attendu.
**RMN** (250MHz, CDCl₃) : 1,24 (d, 3H, OCH₂CH₃) ; 4,12 (q, 2H, OCH₂CH₃) ; 4,36-4,40 (m, 1H, H1 en position alpha ou bêta) ; 4,69-4,92 (m, 1H, H9 en position alpha) ; 7,70 - 7,86 H aromatiques.

### Stade e1 : cyclisation avec POCl₃

- (1S-cis)-9-(1,3-dihydro-1,3-dioxo-2H-isoindol-2-yl)-3,4,7,8,9,10-hexahydro-6,10-dioxo-6H-pyridazino-[1,2-a][1,2]diazepine-1-carboxylate d'éthyle.
- (1R-trans)-9-(1,3-dihydro-1,3-dioxo-2H-isoindol-2-yl)-3,4,7,8,9,10-hexahydro-6,10-dioxo-6H-pyridazino-[1,2-a][1,2]diazepine-1-carboxylate d'éthyle.

A une solution de 20 ml de dichloroéthane chauffée préalablement à 75°C on ajoute en 3 heures et simultanément les solutions A et B suivantes :
A : 417 mg de l'ester préparé au stade d dans 4 ml de dichloroéthane auquel on a ajouté 1 ml d'une solution de 1,2 ml de 2,6-lutidine dans 5 ml de dichloroéthane.
B : 1 ml d'une solution de 1,9 ml de POCl₃ dans 10 ml de dichloroéthane, puis on agite 1 heure à cette température. On refroidit à 10°C, ajoute de l'eau déminéralisée, extrait avec du dichlorométhane et évapore sous pression réduite pour obtenir un produit brut (0,415g) que l'on purifie par chromatographie sur silice en éluant avec le mélange heptane/dichlorométhane-/acétate d'éthyle 1/1/1. On isole 161,8 mg du diastéréo-isomère SS, 126,7 mg du diastéréoisomère SR et 5,8 mg du mélange SS + SR.

### Stade e2 : cyclisation avec POBr₃

- (1S-cis)-9-(1,3-dihydro-1,3-dioxo-2H-isoindol-2-yl)-3,4,7,8,9,10-hexahydro-6,10-dioxo-6H-pyridazino-[1,2-a][1,2]diazepine-1-carboxylate d'éthyle.
- (1R-trans)-9-(1,3-dihydro-1,3-dioxo-2H-isoindol-2-yl)-3,4,7,8,9,10-hexahydro-6,10-dioxo-6H-pyridazino-[1,2-a][1,2]diazepine-1-carboxylate d'éthyle.

A une solution de 20 ml de dichloroéthane chauffée préalablement à 80°C on ajoute en 3 heures et simultanément les solutions A et B suivantes :
A : 417 mg de l'ester préparé au stade d dans 4 ml de dichloroéthane auquel on a ajouté 1 ml d'une solution de 2,4 ml de 2,6-lutidine dans 10 ml de dichloroéthane.
B : 1 ml d'une solution de 5,85 g de POBr₃ dans 10 ml de dichloroéthane, puis on agite 1 heure à cette température. On refroidit à 10°C, ajoute de l'eau déminéralisée, extrait avec du dichlorométhane et évapore sous pression réduite pour obtenir un produit brut (0,419 g) que l'on purifie par chromatographie sur silice en éluant avec le mélange heptane/dichlorométhane/acétate d'éthyle 1/1/1. On isole 163 mg du diastéréoisomère SS, 143 mg du diastéréoisomère SR et 6,2 mg du mélange SS + SR.

### Stade f : déracémisation/épimérisation

- (1S-cis)-9-(1,3-dihydro-1,3-dioxo-2H-isoindol-2-yl)-3,4,7,8,9,10-hexahydro-6,10-dioxo-6H-pyridazino-[1,2-a][1,2]diazepine-1-carboxylate d'éthyle.

On introduit à une température de -45/-48°C en une heure 30 minutes, une solution renfermant 0,029 g de terbutylate de potassium et 0,3 ml de diméthylformamide dans un mélange renfermant 0,194 g du mélange SS + SR préparé au stade d, 1,5 ml de diméthylformamide et 0,75 ml de terbutanol. On maintient le mélange sous agitation pendant 1 heure et introduit après refroidissement à -50°C, 0,4 g de chlorure d'ammonium en poudre. On agite 10 minutes à -45°C, ajoute 1 ml de chlorure d'ammonium à 20°C et agite à nouveau 10 minutes. On ajoute au bout de 5 minutes, 2 ml d'eau déminéralisée. On extrait à l'acétate d'éthyle, lave à l'eau déminéralisée, décante, concentre et sèche. On obtient 0,166 g de diastéréoisomère SS attendu.
α_{D} = -75,3° (1% dans le méthanol)
**RMN** (250MHz, CDCl₃) : 1,73 (m, 3H, H-2alpha H-3alpha H-3bêta; 1,24 (d, 3H, OCH₂CH₃) ; 2,38 (m, 3H, H2bêta, H7alpha, H8 alpha) ; 2,92 (m, 1H, H4alpha) ; 3,39 - 3,44 (m, 1H, H8bêta); 3,62(m, 1H, H7bêta) ; 4,23 (m, 2H, OCH₂CH₃) ; 4,66-4,71 (m, 1H, H4 en position bêta) ; 5,26-5,41 (m, 2H, H1 et H9 en position alpha) ; 7,72 - 7,88 H aromatiques.

## Revendications

1. Procédé de préparation des composés de formule (I) 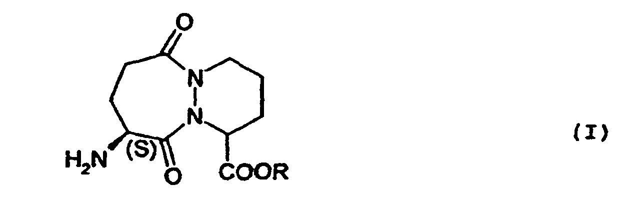 dans laquelle R représente un atome d'hydrogène, un radical alkyle, aryle ou aralkyle renfermant jusqu'à 18 atomes de carbone et la fonction amine peut être libre ou protégée, à partir d'un composé de formule (IA) 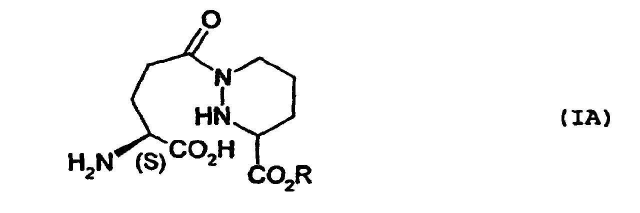 dans laquelle R à la même signification que précédemment et la fonction amine peut être libre ou protégée,
**caractérisé en ce que** la cyclisation de (IA) s'effectue en milieu basique et en présence :
- d'un dérivé d'acide phosphonique de formule (P1) :
P(=Z) (X¹) (X²) (X³)
dans lequel Z est un atome de soufre ou d'oxygène, X¹ représente un atome d'halogène, X² et X³ identiques ou différents représentent un atome d'halogène, un radical alkyloxy renfermant de 1 à 6 atomes de carbone, un radical aryloxy renfermant de 6 à 12 atomes de carbones ou un radical arylalkyloxy renfermant de 7 à 15 atomes de carbone,
- ou d'un trimère de formule (P2) :

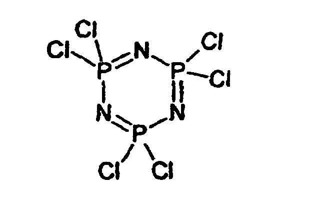

2. Procédé tel que défini à la revendication 1 dans lequel (P1) est le POCl₃.

3. Procédé tel que défini à la revendication 1 dans lequel (P1) est le POBr₃.

4. Procédé tel que défini à l'une quelconque des revendications 1 à 3 dans lequel la base est choisie parmi la pyridine ou la 2,6-lutidine.

5. Procédé tel que défini à l'une quelconque des revendications 1 à 4 **caractérisé en ce que** la température de cyclisation est comprise entre 70 et 80 °C.

6. Procédé tel que défini à l'une quelconque des revendications 1 à 4 **caractérisé en ce que** le solvant est le dichloroéthane.

7. Procédé de préparation du composé de formule (I) sous forme racémique ou optiquement actif (Iopt), comprenant le procédé de cyclisation tel que décrit à la revendication 1, et **caractérisé en ce qu'**il comprend les étapes successives suivantes :
a) On soumet un composé de formule (II) 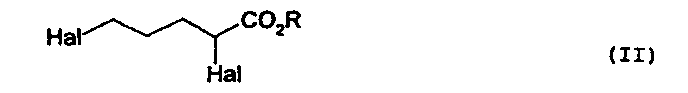 dans laquelle R est tel que défini à la revendication 1 et Hal représenté un atome d'halogène, à l'action d'un composé de formule (III) 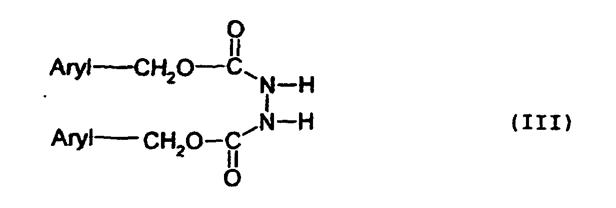 dans laquelle Aryl représente un radical aryle renfermant jusqu'à 14 atomes de carbone, pour obtenir le composé de formule (IV) 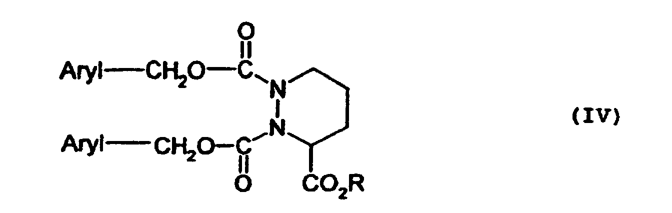 sous forme d'un mélange de stéréoisomères R et S
b) on soumet le composé de formule (IV) à l'action d'un anhydride de formule (F) 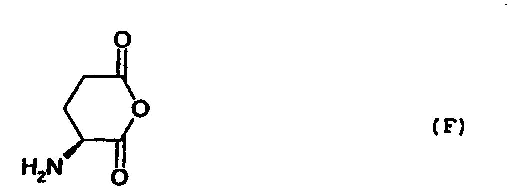 la fonction amine étant sous forme protégée ou non protégée, tout en effectuant une déprotection par hydrogènolyse, afin d'obtenir le composé de formule (IA) 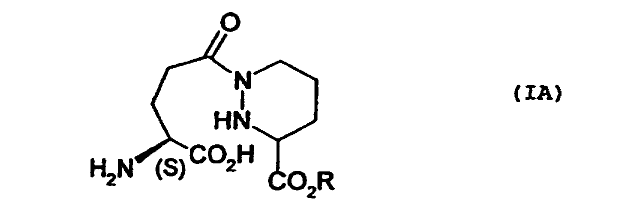 telle que définie à la revendication 1,
c) on soumet selon la revendication 1, le composé de formule (IA) à l'action d'un dérivé d'acide phosphonique (P1) ou (P2) selon les revendications 1 à 3, en présence d'une base, selon la revendication 4, afin d'obtenir le composé de formule (I) tel que défini à la revendication 1 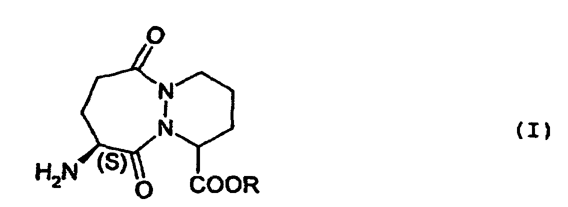
d) le cas échéant, on déprotège la fonction amine pour obtenir le composé de formule (I)dans lequel la fonction amine est non protégée.
e) on soumet le cas échéant le composé de formule (I) sous la forme SS + SR ou SR à l'action d'un agent de déracémisation et/ou épimérisation afin d'obtenir le composé de formule (Iopt) correspondant au diastéréoisomère SS, 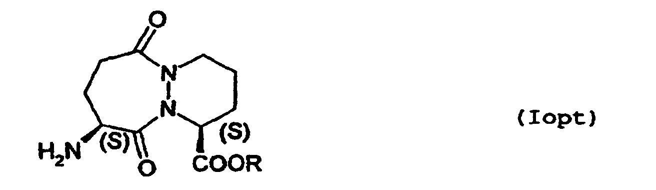
f) le cas échéant, on déprotège la fonction amine pour obtenir le composé de formule (Iopt)dans lequel la fonction amine est non protégée.

8. Procédé tel que défini à la revendication 7 dans lequel F est l'anhydride N-phtaloyl-L-glutamique :

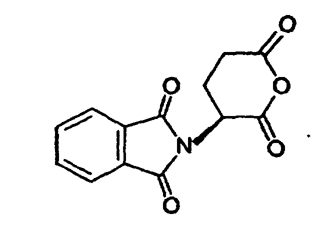

9. Procédé tel que défini à l'une quelconque des revendications 1 à 8, dans lequel la fonction amine des composés de formules (IA), (I) et (Iopt) est protégée sous la forme phtalimido.

10. Procédé tel que défini à l'une quelconque des revendications 1 à 8, dans lequel, au sein des composés de formule (II), (IV), (IA), (I) et (Iopt), R est un radical méthyle, éthyle ou tertbutyle.

11. Procédé tel que défini à l'une quelconque des revendications 1 à 8 dans lequel le composé de formule (I) est le 9-(1,3-dihydro-1,3-dioxo-2H-isoindol-2-yl)-3,4,7,8,9,10-hexahydro-6,10-dioxo-6H-pyridazino-[1,2-a][1,2]diazepine-1-carboxylate d'éthyle :

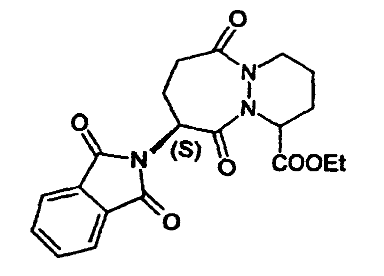

12. Procédé tel que défini à l'une quelconque des revendications 1 à 8 dans lequel le composé de formule (Iopt) est le (1S-cis)-9-(1,3-dihydro-1,3-dioxo-2H-isoindol-2-yl)-3,4,7,8,9,10-hexahydro-6,10-dioxo-6H-pyridazino-[1,2-a][1,2]diazepine-1-carboxylate d'éthyle :

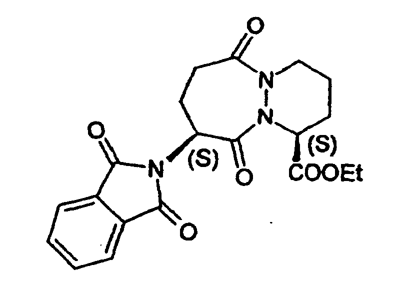

13. Application du procédé tel que défini à l'une quelconque des revendications 1 à 12 comme étape intermédiaire pour la préparation d'un composé de formule (V) 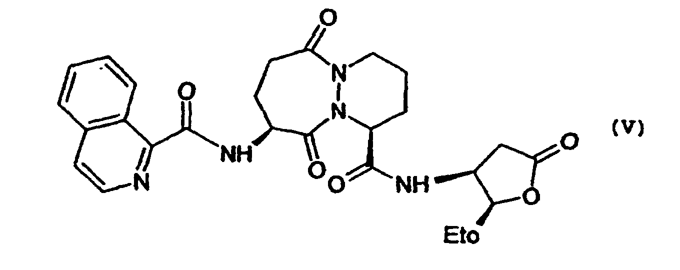 via le composé de formule (Iopt) tel que défini à la revendication 7.

14. Application selon la revendication 13 **caractérisé en ce que** le composé de formule (Iopt) est le (1S-cis)-9-(1,3-dihydro-1,3-dioxo-2H-isoindol-2-yl)-3,4,7,8,9,10-hexahydro-6,10-dioxo-6H-pyridazino-[1,2-a][1,2]diazepine-1-carboxylate d'éthyle.

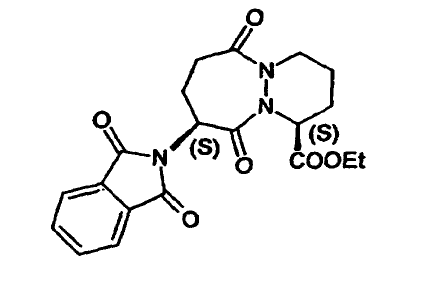

15. Application du procédé tel que défini à l'une quelconque des revendications 1 à 6 comme étape intermédiaire du procédé global de préparation des composés de formules (I) et (Iopt) tels que défini à l'une quelconque des revendications 7 à 14.

16. Composé de formule (IA) telle que définie à la revendication 1.

## Patentansprüche

1. Verfahren zur Herstellung von Verbindungen der Formel (I) 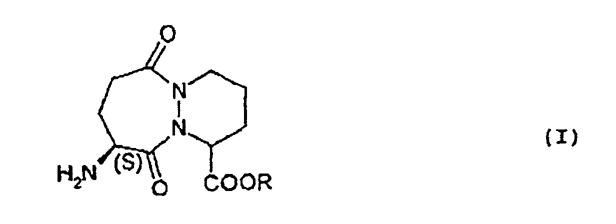 in der R ein Wasserstoffatom, einen Alkyl-, Aryl- oder Aralkylrest, der bis zu 18 Kohlenstoffatome umfasst, darstellt und die Aminfunktion frei oder geschützt sein kann, ausgehend von einer Verbindung der Formel (IA): 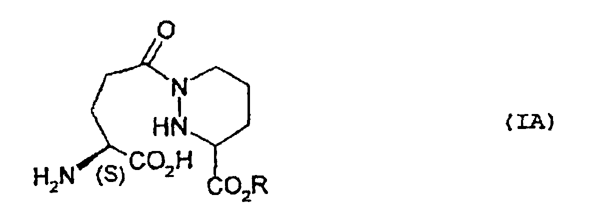 in der R dieselbe Bedeutung wie vorstehend hat und die Aminfunktion frei oder geschützt sein kann,
**dadurch gekennzeichnet, dass**
die Cyclisierung von (IA) in basischem Milieu und in Gegenwart
- eines Phosphonsäurederivats der Formel (P1):
P(=Z) (X¹) (X²) (X³)
in der Z ein Schwefel- oder Sauerstoffatom ist; X¹ ein Halogenatom darstellt; X² und X³, die gleich oder unterschiedlich sind, ein Halogenatom, einen Alkyloxyrest, der 1 bis 6 Kohlenstoffatome enthält, einen Aryloxyrest, der 6 bis 12 Kohlenstoffatome enthält, oder einen Arylalkyloxyrest, der 7 bis 15 Kohlenstoffatome enthält, darstellen;
- oder eines Trimers der Formel (P2) 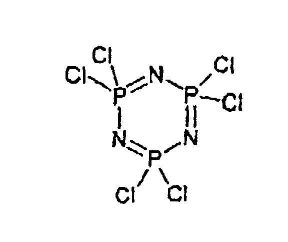
erfolgt.

2. Verfahren nach Anspruch 1, wobei (P1) POCl₃ ist.

3. Verfahren nach Anspruch 1, wobei (P1) POBr₃ ist.

4. Verfahren nach einem der Ansprüche 1 bis 3, wobei die Base unter Pyridin und 2,6-Lutidin ausgewählt wird.

5. Verfahren nach einem der Ansprüche 1 bis 4, **dadurch gekennzeichnet, dass** die Cyclisierungstemperatur zwischen 70 und 80°C liegt.

6. Verfahren nach einem der Ansprüche 1 bis 4, **dadurch gekennzeichnet, dass** das Lösungsmittel Dichlorethan ist.

7. Verfahren zur Herstellung der Verbindung der Formel (I) in racemischer Form oder optisch-aktiver Form (Iopt), das das Cyclisierungsverfahren, wie es in Anspruch 1 beschrieben ist, umfasst und das **dadurch gekennzeichnet, ist, dass** es die folgenden aufeinanderfolgenden Stufen umfasst:
a) man unterwirft eine Verbindung der Formel (II) 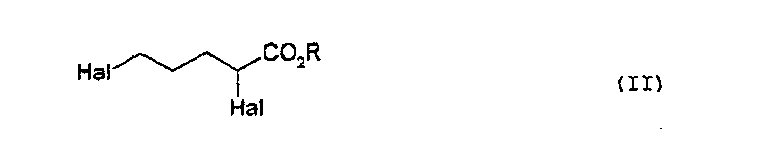 in der R wie oben in Anspruch 1 definiert ist und Hal ein Halogenatom darstellt, der Einwirkung einer Verbindung der Formel (III) 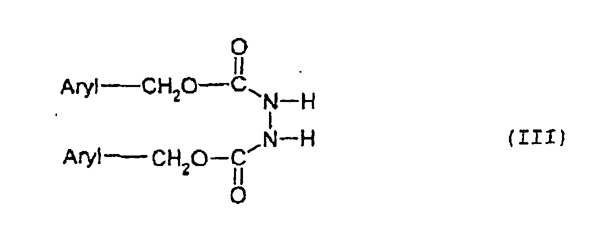 in der Aryl einen Arylrest darstellt, der bis zu 14 Kohlenstoffatome enthält, um die Verbindung der Formel (IV) 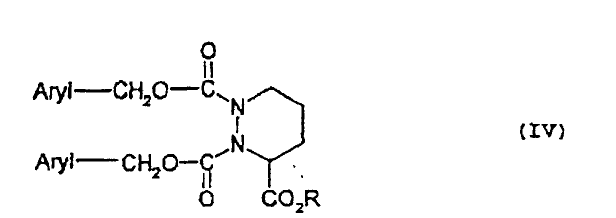 in Form eines Gemisches der Stereoisomeren R und S zu erhalten;
b) man unterwirft die Verbindung der Formel (IV) der Einwirkung eines Anhydrids der Formel (F) 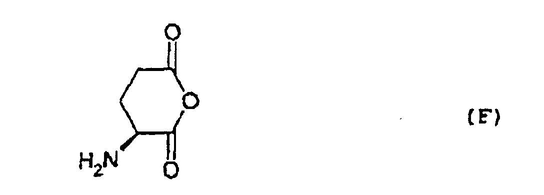 wobei die Aminfunktion in geschützter oder nicht geschützter Form vorliegt, wobei eine Entschützung durch Hydrogenolyse durchgeführt wird, um die Verbindung der Formel (IA) 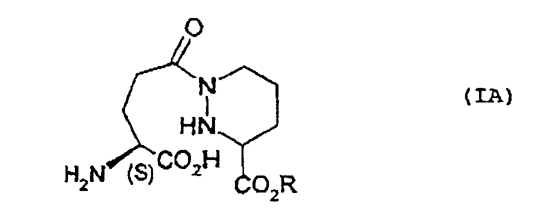 wie sie in Anspruch 1 definiert ist, zu erhalten;
c) man unterwirft gemäß Anspruch 1 die Verbindung der Formel (IA) der Wirkung eines Phosphonsäurederivats (P1) oder (P2) nach den Ansprüchen 1 bis 3 in Gegenwart einer Base, nach Anspruch 4, um die Verbindung der Formel (I), wie sie in Anspruch 1 definiert ist, zu erhalten 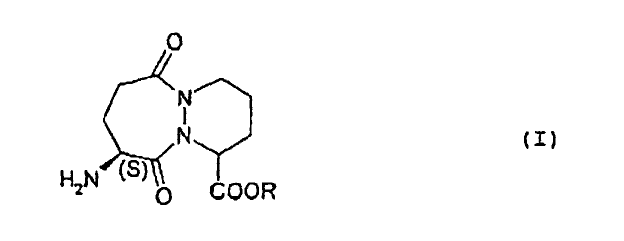
d) gegebenenfalls entschützt man die Aminfunktion unter Erhalt der Verbindung der Formel (I), in der die Aminfunktion nicht geschützt ist;
e) gegebenenfalls unterwirft man die Verbindung der Formel (I) in der Form SS + SR oder SR der Einwirkung des Entracemisierungs- und/oder Epimerisierungsmittels, um die Verbindung der Formel (Iopt) zu erhalten, die dem Diastereoisomer SS entspricht, 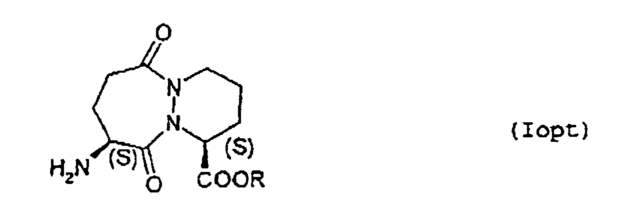
f) gegebenenfalls entschützt man die Aminfunktion, um die Verbindung der Formel (Iopt), in der die Aminfunktion nicht geschützt ist, zu erhalten.

8. Verfahren nach Anspruch 7, wobei F N-Phthaloyl-Lglutaminsäureanhydrid ist:

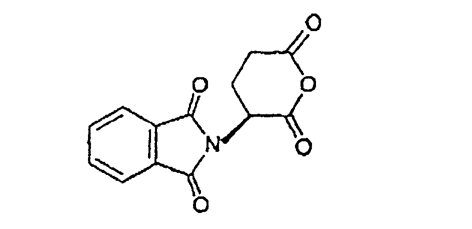

9. Verfahren nach einem der Ansprüche 1 bis 8, wobei die Aminfunktion der Verbindungen der Formeln (IA), (I) und (Iopt) in der Phthalimidoform geschützt ist.

10. Verfahren nach einem der Ansprüche 1 bis 8, wobei in den Verbindungen der Formel (II), (IV), (IA), (I) und (Iopt) R ein Methyl-, Ethyl- oder tert-Butyl-Rest ist.

11. Verfahren nach einem der Ansprüche 1 bis 8, wobei die Verbindung der Formel (I) 9-(1,3-Dihydro-1,3-dioxo-2Hisoindol-2-yl)-3,4,7,8,9,10-hexahydro-6,10-dioxo-6Hpyridazino-[1,2-a][1,2]diazepin-1-carbonsäureethylester ist:

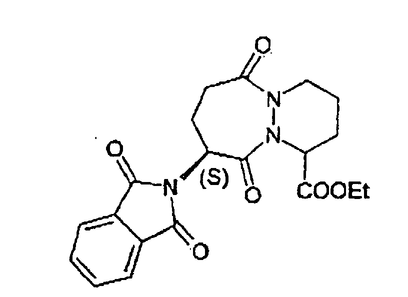

12. Verfahren nach einem der Ansprüche 1 bis 8, wobei die Verbindung der Formel (Iopt) (1S-cis)-9-(1,3-Dihydro-1,3-dioxo-2H-isoindol-2-yl)-3,4,7,8,9,10-hexahydro-6,10-dioxo-6H-pyridazino-[1,2-a][1,2]diazepin-1-carbonsäureethylester ist:

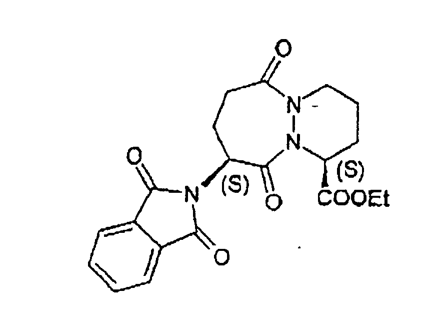

13. Anwendung des Verfahrens, wie es in einem der Ansprüche 1 bis 12 definiert ist, als Zwischenstufe für die Herstellung einer Verbindung der Formel (V) 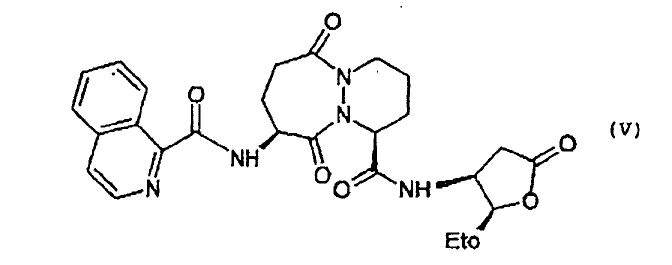 über die Verbindung der Formel (Iopt), wie sie in Anspruch 7 definiert ist.

14. Anwendung nach Anspruch 13, **dadurch gekennzeichnet, dass** die Verbindung der Formel (Iopt) (1S-cis)-9-(1,3-Dihydro-1,3-dioxo-2H-isoindol-2-yl)-3,4,7,8,9,10-hexahydro-6,10-dioxo-6H-pyridazino-[1,2-a][1,2]-diazepin-1-carbonsäureethylester ist:

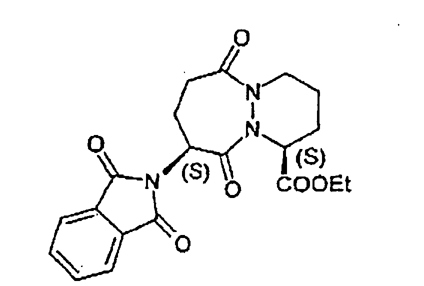

15. Anwendung des Verfahrens nach einem der Ansprüche 1 bis 6 als Zwischenstufe des allgemeinen Verfahrens zur Herstellung der Verbindungen der Formeln (I) und (Iopt), wie sie in einem der Ansprüche 7 bis 14 definiert sind.

16. Verbindung der Formel (IA), wie sie in Anspruch 1 definiert ist.

## Claims

1. Process for preparing the compounds of formula (I) 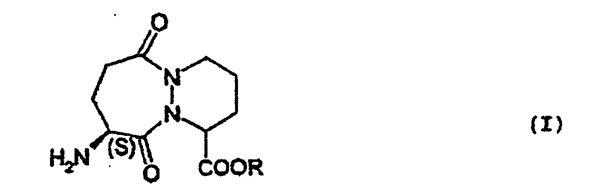 in which R represents a hydrogen atom or an alkyl, aryl or aralkyl radical containing up to 18 carbon atoms, and the amine function may be free or protected, from a compound of formula (IA) 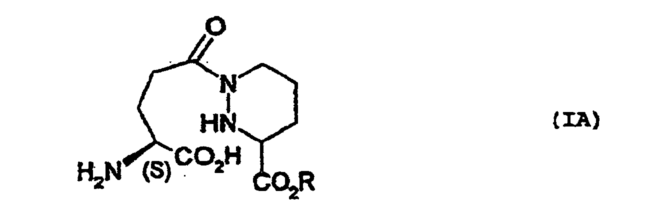 in which R has the same meaning as above and the amine function may be free or protected,
**characterized in that** the cyclization of (IA) is carried out in basic medium and in the presence:
- of a derivative of phosphonic acid of formula (P1) :
P(=Z) (X¹) (X²) (X³)
in which Z is a sulphur or oxygen atom, X¹ represents a halogen atom, and X² and X³, which may be identical or different, represent a halogen atom, an alkyloxy radical containing from 1 to 6 carbon atoms, an aryloxy radical containing from 6 to 12 carbon atoms or an arylalkyloxy radical containing from 7 to 15 carbon atoms,
- or of a trimer of formula (P2):

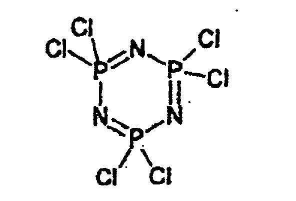

2. Process as defined in Claim 1, in which (P1) is POCl₃.

3. Process as defined in Claim 1, in which (P1) is POBr₃.

4. Process as defined in any one of Claims 1 to 3, in which the base is chosen from pyridine and 2,6-lutidine.

5. Process as defined in any one of Claims 1 to 4, **characterized in that** the cyclization temperature is between 70 and 80°C.

6. Process as defined in any one of Claims 1 to 4, **characterized in that** the solvent is dichloroethane.

7. Process for preparing the compound of formula (I) in racemic or optically active form (Iopt), comprising the cyclization process as described in Claim 1, and **characterized in that** it comprises the following successive steps:
a) a compound of formula (II) 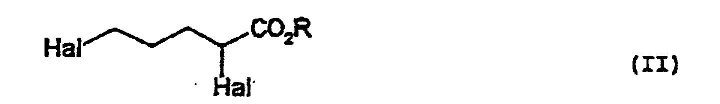 in which R is as defined in Claim 1 and Hal represents a halogen atom, is subjected to the action of a compound of formula (III) 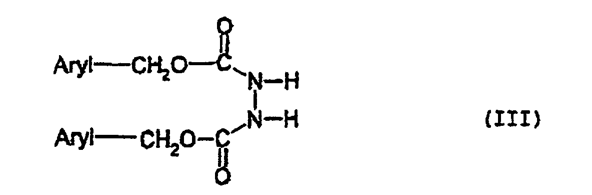 in which Aryl represents an aryl radical containing up to 14 carbon atoms, so as to obtain the compound of formula (IV) 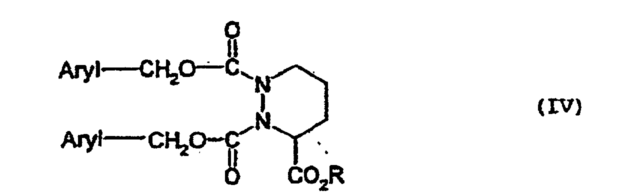 in the form of a mixture of R and S stereoisomers;
b) the compound of formula (IV) is subjected to the action of an anhydride of formula (F) 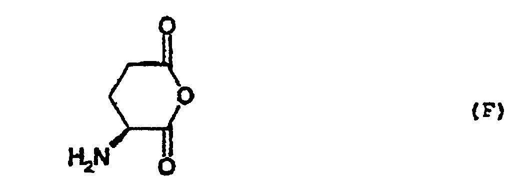 the amine function being in protected or unprotected form, while at the same time carrying out a deprotection by hydrogenolysis,
in order to obtain the compound of formula (IA) 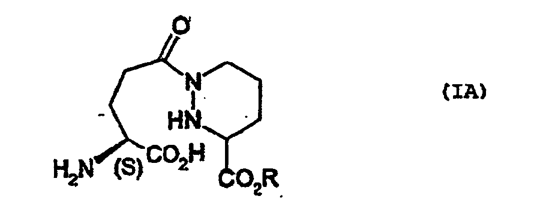 as defined in Claim 1;
c) the compound of formula (IA) is subjected, according to Claim 1, to the action of a derivative of phosphonic acid (P1) or (P2) according to Claims 1 to 3, in the presence of a base, according to Claim 4, in order to obtain the compound of formula (I) as defined in Claim 1 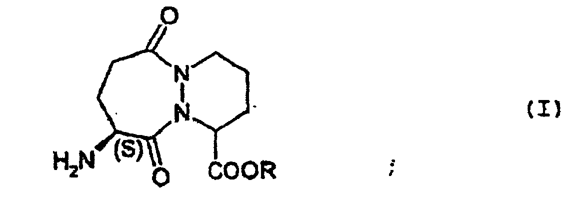
d) where appropriate, the amine function is deprotected so as to obtain the compound of formula (I) in which the amine function is unprotected;
e) the compound of formula (I) in the SS + SR or SR form is subjected, where appropriate, to the action of a deracemization and/or epimerization agent in order to obtain the compound of formula (Iopt) corresponding to the SS diastereoisomer 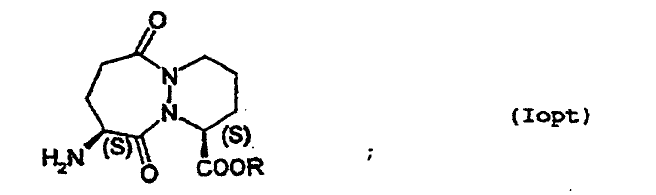
f) where appropriate, the amine function is deprotected so as to obtain the compound of formula (Iopt) in which the amine function is unprotected.

8. Process as defined in Claim 7, in which F is N-phthaloyl-L-glutamic anhydride:

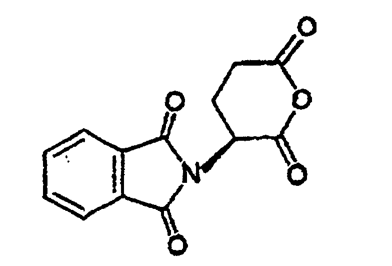

9. Process as defined in any one of Claims 1 to 8, in which the amine function of the compounds of formulae (IA), (I) and (Iopt) is protected in the phthalimido form.

10. Process as defined in any one of Claims 1 to 8, in which, within the compounds of formula (II), (IV), (IA), (I) and (Iopt), R is a methyl, ethyl or tert-butyl radical.

11. Process as defined in any one of Claims 1 to 8, in which the compound of formula (I) is ethyl 9-(1,3-dihydro-1,3-dioxo-2H-isoindol-2-yl)-3,4,7,8,9,10-hexahydro-6,10-dioxo-6H-pyridazino[1,2-a][1,2]diazepine-1-carboxylate:

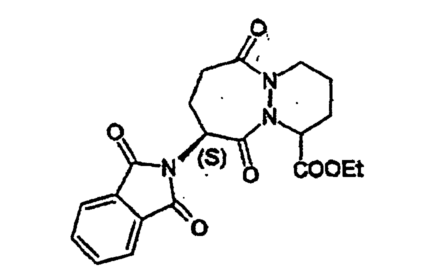

12. Process as defined in any one of Claims 1 to 8, in which the compound of formula (Iopt) is ethyl (1S-cis)-9-(1,3-dihydro-1,3-dioxo-2H-isoindol-2-yl)-3,4,7,8,9,10-hexahydro-6,10-dioxo-6H-pyridazino[1,2-a]-[1,2]diazepine-1-carboxylate:

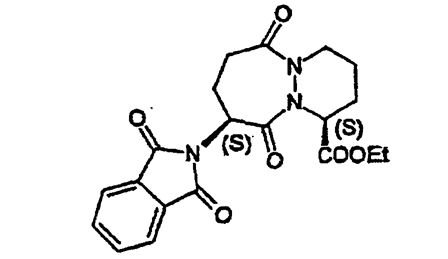

13. Application of the process as defined in any one of Claims 1 to 12, as an intermediate step for preparing a compound of formula (V) 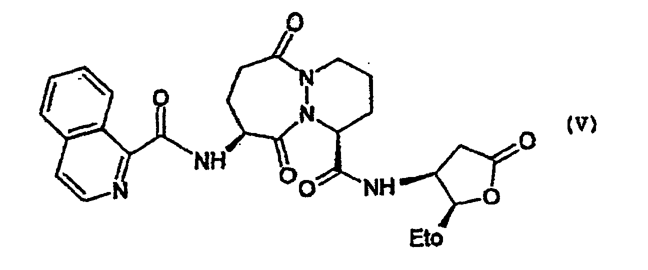 via the compound of formula (Iopt) as defined in Claim 7.

14. Application according to Claim 13, **characterized in that** the compound of formula (Iopt) is ethyl (1S-cis)-9-(1,3-dihydro-1,3-dioxo-2H-isoindol-2-yl)-3,4,7,8,9,10-hexahydro-6,10-dioxo-6H-pyridazino[1,2-a]-[1,2]diazepine-1-carboxylate

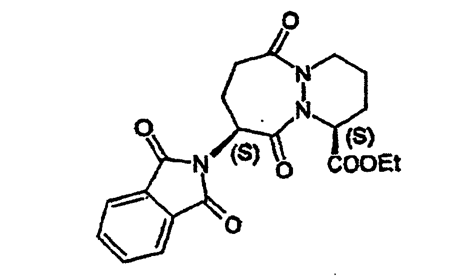

15. Application of the process as defined in any one of Claims 1 to 6, as an intermediate step in the overall process for preparing the compounds of formulae (I) and (Iopt) as defined in any one of Claims 7 to 14.

16. Compound of formula (IA) as defined in Claim 1.
